# EUROPÄISCHE PATENTSCHRIFT

(11) **EP 3 011 091 B1**
(45) Veröffentlichungstag und Bekanntmachung des Hinweises auf die Patenterteilung: **08.03.2017**
(21) Anmeldenummer: 14747795.4
(22) Anmeldetag: 13.06.2014
(51) Int. Cl.: D01F 9/00, D01F 2/06, C08B 37/00, C08L 5/00

(54) **POLYSACCHARIDFASER UND VERFAHREN ZU IHRER HERSTELLUNG**
POLYSACCHARIDE FIBRES AND METHOD FOR PRODUCING SAME
FIBRES DE POLYSACCHARIDE ET LEUR PROCÉDÉ DE PRODUCTION

(30) Priorität: 17.06.2013 AT 4842013
(43) Veröffentlichungstag der Anmeldung: 27.04.2016
(73) Patentinhaber: Lenzing AG, 4860 Lenzing (AT)
(72) Erfinder: RÖDER, Thomas, 4840 Vöcklabruck (AT); KAINDL, Gernot, 4860 Lenzing (AT); REDLINGER, Sigrid, 4860 Lenzing (AT); FIRGO, Heinrich, 4840 Vöcklabruck (AT); KRONER, Gert, 4863 Seewalchen (AT)
(74) Vertreter: Hanemann, Otto
(86) Internationale Anmeldenummer: PCT/AT2014/000124
(87) Internationale Veröffentlichungsnummer: WO 2014/201483

(56) Entgegenhaltungen:
- WO-A1-97/04148
- WO-A1-98/55673
- WO-A1-2013/052730
- DE-A1- 3 036 415

## Beschreibung

Die vorliegende Erfindung betrifft ein Verfahren zur Herstellung von Polysaccharidfasern, die als faserbildende Substanz eine Mischung von Cellulose und α(1→3)-Glucan enthalten sowie die daraus hergestellten Fasern und ihre Verwendung.

### Stand der Technik

Polysaccharide spielen als Materialien, die aus nachwachsenden Rohstoffen gewonnen werden können, eine immer größere Rolle. Eines der am häufigsten vorkommenden Polysaccharide ist die Cellulose. Baumwollfasern, die fast ausschließlich aus Cellulose bestehen, sind ein Beispiel für die Wichtigkeit von Polysacchariden. Aber auch aus anderen cellulosischen Rohstoffen gewonnene Materialien, wie z. B. cellulosische Kunstfasern, gewinnen immer mehr an Bedeutung.

Die Gattungsnamen "Viscose-Fasern" und "Modat-Fasern" wurden von der BISFA (Bureau for the International Standardization of Man-Made-Fibers) Cellulosefasern zugeteilt, die durch chemische Derivatisierung von Cellulose mithilfe von Natronlauge und Schwefelkohlenstoff hergestellt werden.

Der Name "Modatfaser" ist ein generischer Begriff, der gemäß der Definition der BISFA für eine Cellulosefaser mit einer definierten hohen Nassfestigkeit und einem ebenfalls definierten hohen Nassmodul (d.h. die Kraft, welche benötigt wird, um die Faser in nassem Zustand um 5% zu dehnen) steht. Das Modal-Verfahren kann als Abwandlung des Viskose-Verfahrens angesehen werden..

Viscose- und Modal-Verfahren sollen für die Zwecke der vorliegenden Erfindung auch zusammenfassend als "Xanthogenatverfahren" bezeichnet werden, da in ihnen stets Polysaccharide mit CS2 zu den entsprechenden Xanthogenaten umgesetzt werden. Xanthogenat-Verfahren zur Herstellung von Cellulosefasern sind dem Fachmann seit Jahrzehnten grundsätzlich bekannt. Ein Verfahren zur Herstellung von Modalfasern ist beispielsweise aus AT 287.905 B bekannt.

Der in Xanthogenatverfahren-Verfahren hauptsächlich verwendete cellulosische Rohstoff ist Zellstoff, der aus Holz gewonnen wird. Die im Holz, wie auch in anderen pflanzlichen Cellulosequellen wie Baumwoll-Linters, Stroh, etc. vorliegenden Cellulosemoleküle sind sehr langkettig, d. h. sie weisen einen hohen Polymerisationsgrad auf. Um eine großtechnisch gut verarbeitbare Cellulosespinnlösung zu erhalten, muss der Polymerisationsgrad der Cellulosemoleküle gezielt eingestellt werden, wobei zwangsläufig ein Teil der Polymermoleküle verkürzt werden. Dies geschieht in den üblichen Zellstoffherstellungsverfahren sowie auch in separaten Vorbehandlungsstufen wie Bleichen, Säurebehandlung oder Bestrahlung durch eine Spaltung der ursprünglich langen Cellulosemoleküle. Neben den kürzeren Ketten mit dem angestrebten Polymerisationsgrad entstehen dabei aber auch noch wesentlich kürzere Bruchstücke wie Oligomere oder sogar Monomere, die spätestens beim Ausfällen der Spinnlösung im Fällbad in Lösung bleiben, nicht zur Faserbildung beitragen und damit verloren gehen. Die auf diesem Weg eintretenden Rohstoffverluste können erheblich sein und die Wirtschaftlichkeit des gesamten Verfahrens beeinträchtigen.

Die US 7,000,000 beschreibt Fasern, die durch Verspinnen einer Lösung von Polysacchariden, die im Wesentlichen aus Hexose-Wiederholungseinheiten bestehen, die über α(1→3)-glycosidische Bindungen verknüpft sind, erhalten werden. Diese Polysaccharide können hergestellt werden, indem eine wässrige Lösung von Saccharose mit Glucosyltransferase (GtfJ), isoliert aus Streptococcus salivarius, in Kontakt gebracht wird (Simpson et al. Microbiology, vol 41, pp 1451-1460 (1995)). "Im Wesentlichen" bedeutet in diesem Zusammenhang, dass innerhalb der Polysaccharidketten vereinzelt Fehlstellen auftreten können, an denen andere Bindungskonfigurationen auftreten. Diese Polysaccharide sollen für die Zwecke der vorliegenden Erfindung als "α(1→3)-Glucan" bezeichnet werden.

Die US 7,000,000 offenbart zunächst Möglichkeiten zur enzymatischen Herstellung von α(1→3)-Glucan aus Monosacchariden. Auf diese Weise können relativ kurzkettige Polysaccharide ohne Verlust an Monomerbausteinen hergestellt werden, da die Polymerketten aus den Monomerbausteinen aufgebaut werden. Im Gegensatz zur Herstellung kurzkettiger Cellulosemoleküle ist die Herstellung von α(1→3)-Glucan umso preisgünstiger, je kürzer die Polymerketten sind, da dann nur eine geringe Verweilzeit in den Reaktoren notwendig ist.

Gemäß der US 7,000,000 soll das α(1→3)-Glucan derivatisiert, bevorzugt acetyliert, werden. Das Lösungsmittel ist bevorzugt eine organische Säure, eine organische Halogenverbindung, ein fluorierter Alkohol oder eine Mischung aus solchen Komponenten. Diese Lösungsmittel sind teuer und aufwendig zu regenerieren.

Die WO 98/55673 A1 offenbart ein Verfahren zur Herstellung einer Polysaccharid-Faser nach einem Xanthogenat-Verfahren, dadurch gekennzeichnet, dass die faserbildende Substanz eine Mischung aus Cellulose und Polygalactomannan ist.

Die WO 97/04148 A1 offenbart ein Verfahren zur Herstellung von modifizierten Viskosefasern durch Mischen einer Lösung von Cellulosecarbamat mit einer Lösung von Cellulosexanthogenat. Dieses Verfahren ist sehr aufwendig und komplex, da hierbei zwei Cellulosederivate kombiniert werden, die jeweils zunächst aus Cellulose hergestellt und später jeweils wieder in reine Cellulose überführt werden müssen, wobei dann zwei Arten von abgespaltenen Nebenprodukten anfallen.

Die DE 30 36 415 A1 offenbart Mischfasern auf Basis regenerierter Cellulose mit anionisch modifizierten Polysacchariden als Mischkomponente, wodurch die Fasern hohe Saugfähigkeit und hohes Flüssigkeitsrückhaltevermögen aufweisen. Die anionisch modifizierten Polysaccharide werden in eine Spinnviskose eingemischt. Hierbei handelt es sich um also um Spezialfasern mit sehr speziellen Eigenschaften und begrenztem Anwendungsgebiet.

Die WO 2013/052730 A1 offenbart Fasern mit α(1→3)-Glucan als faserbildende Substanz, die nach dem sogenannten Aminoxid-Verfahren mit NMMO als Lösungsmittel durch Verspinnen hergestellt wurden. Das Aminoxid-Verfahren ist grundsätzlich anders gestaltet als das Viscose- oder das Modal-Verfahren und erfordert eine völlig andere Produktionsanlage. Eine Viscose-Produktionsanlage kann nicht durch einfache Umbaumaßnahmen auf das Aminoxid-Verfahren umgestellt werden.

Es wurde daher versucht, α(1→3)-Glucane anstelle von Cellulose in einem Viskose- oder Modal-Verfahren unter großtechnisch kommerziell angewendeten Verfahrensbedingungen einzusetzen. Leider zeigte sich, dass sich unter diesen Bedingungen α(1→3)-Glucane nicht zufriedenstellend zu Fasern verarbeiten ließen, da sich Glucane in verdünnter Natronlauge lösen. Aufgrund dieser Tatsache können α(1→3)-Glucane in den bestehenden Verfahren nicht einfach anstatt Cellulose eingesetzt werden.

### Aufgabe

Die Aufgabe bestand gegenüber diesem Stand der Technik darin, eine Polysaccharid-Faser sowie ein Verfahren zu deren Herstellung zur Verfügung zu stellen, die nicht die oben genannten Probleme (z.B. der Löslichkeit in Natronlauge) aufweist. Der Polysaccharid-Rohstoff sollte preiswert sein und das Verarbeitungsverfahren sollte bereits großtechnisch etabliert und wirtschaftlich sowie auf vorhandenen Anlagen durchführbar sein.

### Beschreibung der Erfindung

Die Lösung der oben beschriebenen Aufgabe besteht in einem Verfahren zur Herstellung einer Polysaccharid-Faser nach einem Xanthogenatverfahren, wobei die faserbildende Substanz eine Mischung aus Cellulose und α(1→3)-Glucan ist. Das α(1→3)-Glucan kann an verschiedenen Stellen des Prozesses in Form einer α(1→3)-Glucan-haltigen Natronlaugelösung zugesetzt werden. Eine auf diese Weise hergestellte Faser soll für die Zwecke der vorliegenden Erfindung ebenfalls als Viscose- oder Modal-Faser bezeichnet werden, obwohl sie neben Cellulose noch ein weiteres faserbildendes Polysaccharid, nämlich das α(1→3)-Glucan, enthält.

Für die Zwecke der vorliegenden Erfindung soll der Begriff "Faser" sowohl Stapelfasern mit definierter Schnittlänge als auch Endlosfilamente umfassen. Sämtliche im Folgenden beschriebenen Prinzipien der Erfindung gelten grundsätzlich sowohl für Stapelfasern als auch für Endlosfilamente.

Der Einzelfasertiter der erfindungsgemäßen Fasern kann zwischen 0,1 und 10 dtex betragen. Bevorzugt ist er zwischen 0,5 und 6,5 dtex und besonders bevorzugt zwischen 0,9 und 6,0 dtex. Im Falle von Stapelfasern beträgt die Schnittlänge üblicherweise zwischen 0,5 und 120 mm, bevorzugt zwischen 20 und 70 mm und besonders bevorzugt zwischen 35 und 60 mm. Im Falle von Endlosfilamenten beträgt die Anzahl der Einzelfilamente im Filamentgarn zwischen 50 und 10.000, bevorzugt zwischen 50 und 3.000.

Das α(1→3)-Glucan kann hergestellt werden, indem eine wässrige Lösung von Saccharose mit Glucosyltransferase (GtfJ) isoliert aus Streptococcus salivarius in Kontakt gebracht wird (Simpson et al. Microbiology, vol 41, pp 1451-1460 (1995)).

Bevorzugte Ausführungsformen des erfindungsgemäßen Verfahrens sind die dem Fachmann grundsätzlich bekannten Varianten des Viscoseverfahrens sowie eines zur Herstellung von Modalfasern modifizierten Viscoseverfahrens.

In einer bevorzugten Ausführungsform des erfindungsgemäßen Verfahrens besteht das α(1→3)-Glucan zu mindestens 90 % aus Hexose-Einheiten und mindestens 50 % der Hexose-Einheiten sind durch α(1→3)-glycosidische Bindungen verknüpft.

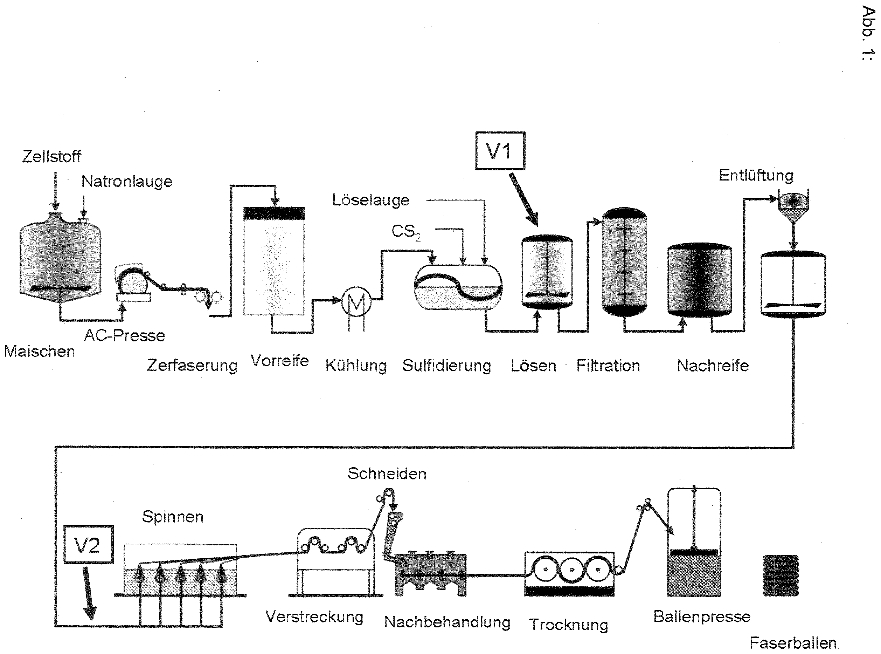

Das Verfahren zur Herstellung der erfindungsgemäßen Faser besteht aus folgenden Abschnitten (siehe auch Abbildung 1):
1. Herstellung der Alkalicellulose und deren Xanthogenierung;
2a. Zugabe von α(1→3) Glucan mit der Löselauge (Abbildung 1, Zugabestelle V1), bevorzugt durch Zugabe in einen entsprechenden Rührbehälter,
   oder
2b. Lösen des Xanthogenats in Löselauge und Zugabe von α(1→3) Glucan in alkalischer Lösung zwischen Löser und Spinnmaschine (Abbildung 1, Zugabestelle V2), bevorzugt durch ein entsprechendes, dem Fachmann bekanntes inline-Mischaggregat;
3. Ausspinnen der α(1→3) Glucan-haltigen Spinnlösung durch eine Düse in ein schwefelsaures Spinnbad, Verstreckung der Fasern und Nachbehandlung.

Die Konzentration der faserbildenden Substanz in der Spinnlösung kann zwischen 4 und 15 Gew.-% betragen, bevorzugt sind 5,5 bis 12 Gew.-%.

Die faserbildende Substanz kann im erfindungsgemäßen Verfahren zwischen 1 und 99 Gew.-% α(1→3)-Glucan enthalten. Besonders bevorzugt ist ein Anteil des α(1→3)-Glucans zwischen 5 und 45 Gew.-%. Unterhalb 5 Gew.-% α(1→3)-Glucans ist der wirtschaftliche Effekt des α(1→3) Glucan-Zusatzes für übliche Anwendungen der erfindungsgemäßen Fasern zu gering; oberhalb 45 Gew.-% werden Konkurrenzreaktionen um das CS2 in der Spinnlösung zu groß und die Spinnbarkeit der Lösung nimmt deutlich ab. Beide Grenzen können jedoch unter besonderen Bedingungen bzw. für besondere Anwendungen der erfindungsgemäßen Fasern überschritten werden; auch Fasern mit einem α(1→3)-Glucan-Anteil-zwischen 1 und 5 Gew.-% bzw. zwischen 45 und 99 Gew.-% sind vom Umfang der vorliegenden Erfindung ausdrücklich mit umfasst.

Der restliche Anteil an der faserbildenden Substanz besteht bevorzugt im Wesentlichen aus Cellulose. "Im Wesentlichen" bedeutet in diesem Zusammenhang, dass geringe Mengen anderer Substanzen enthalten sein können, die vor allem aus dem cellulosischen Rohstoff, im Allgemeinen dem Zellstoff stammen. Solche andere Substanzen sind vor allem Hemicellulose und andere Saccharide, Ligninreste oder Ähnliches. Sie sind auch in handelsüblichen Viskose- und Modalfasern enthalten.

Der Umfang der vorliegenden Erfindung soll jedoch ausdrücklich auch solche Fasern umfassen, die neben den bisher genannten Bestandteilen auch weitere Polysaccharide oder funktionale Additive, wie sie in der Nonwovens-und Textilindustrie allgemein bekannt sind, enthalten.

Der Polymerisationsgrad des im erfindungsgemäßen Verfahren eingesetzten α(1→3) Glucans, ausgedrückt als Gewichtsmittel DP_{w}, kann zwischen 200 und 2000 liegen; bevorzugt sind Werte zwischen 500 und 1000.

Gegenstand der vorliegenden Erfindung ist auch eine nach einem Xanthogenatverfahren hergestellte Polysaccharid-Faser, die als faserbildende Substanz Cellulose und α(1→3)-Glucan enthält. Bevorzugt enthält die faserbildende Substanz zwischen 1 und 99 Gew.-% und besonders bevorzugt zwischen 5 und 45 Gew.-% α(1→3)-Glucan.

In einer bevorzugten Ausführungsform besteht das α(1→3)-Glucan der erfindungsgemäßen Polysaccharid-Faser zu mindestens 90 % aus Hexose-Einheiten und mindestens 50 % der Hexose-Einheiten sind durch α(1→3)-glycosidische Bindungen verknüpft.

Ebenfalls Gegenstand der vorliegenden Erfindung ist die Verwendung der erfindungsgemäßen Fasern zur Herstellung von verschiedensten trocken- und nass gelegten Papieren, Vliesstoffen, Hygieneartikeln wie Tampons, Slipeinlagen und Windeln und sonstigen Vliesstoffen, insbesondere absorbierenden Nonwovens-Produkten, aber auch von textilen Erzeugnissen wie Garnen, Geweben, Gestricken oder Gewirken.

Im Folgenden wird die Erfindung anhand von Beispielen beschrieben. Die Erfindung ist jedoch ausdrücklich nicht auf diese Beispiele beschränkt, sondern umfasst auch alle anderen Ausführungsformen, die auf dem gleichen erfinderischen Konzept beruhen.

### Beispiele

Der Polymerisationsgrad der α(1→3)-Glucane wurde mittels GPC in DMAc/LiCI ermittelt. Im Folgenden wird stets das Gewichtsmittel des Polymerisationsgrades (DP_{w}) angegeben.

### Beispiel 1:

Ein Viskosexanthogenat, enthaltend 29,8 Gew.-% Cellulose, 14,9 Gew.-% Alkali und 8 Gew.-% Schwefel, wurde in einem Löseaggregat mit einer Löselauge 1 enthaltend 4,5 Gew.-% NaOH und danach mit einer Löselauge 2 enthaltend 9 Gew.-% α(1→3)-Glucan und 4,5 Gew.-% NaOH und abschließend mit Wasser umgesetzt. Die so hergestellte Viskose enthält 9 Gew.-% faserbildendes Material, 5,20 Gew.-% Alkali und 2,4 Gew.-% Schwefel (berechnet unter der Annahme von 100 Gew.-% Cellulose als faserbildendes Material), mit einem Reifeindex von 14 Hottenroth und einer Kugelfallviskosität von 80 Sekunden (bestimmt gemäß dem Zellcheming-Merkblatt III/5/E). Es wurden Viskoselösungen mit 10 und 25% α(1→3)-Glucan hergestellt. Diese Glucan-Mengen beziehen sich auf den Anteil des α(1→3)-Glucans an der faserbildenden Substanz. Diese Viskosen enthalten 2,2 Gew.-% Schwefel (10 Gew.-% Glucan und 90 Gew.-% Cellulose als faserbildendes Material) bzw. 1,8 Gew.-% Schwefel (25 Gew.-% Glucan und 75 Gew.-% Cellulose als faserbildendes Material). Die Lösung wurde mittels einer Spinndüse in ein Regenerierbad, enthaltend 100 g/l Schwefelsäure, 330 g/l Natriumsulfat und 15 g/l Zinksulfat, gesponnen. Die Spinndüse hatte 1053 Löcher mit 50µm Durchmesser. Der Viskosespinnlösung wurden 0,5 Gew.-% einen stickstoffhaltigen Hilfsmittels zugesetzt. Zur Erzielung einer entsprechenden Faserfestigkeit erfolgt eine Verstreckung im Zweitbad (92 C, 15 g/l H2S04) von ca. 75%. Die Abzugsgeschwindigkeit beträgt 50 m/min.

In einem Vergleichsbeispiel 1 wurde die Viskose aus Beispiel 1 ohne Zusatz der Glucan-NaOH-Lösung, aber unter ansonsten gleichen Bedingungen wie in Beispiel 1 zu Fasern versponnen.

Die Eigenschaften der erhaltenen Fasern sind in der Tabelle 1 angegeben:

### Beispiel 2:

Eine Viskose, enthaltend 8,70 Gew.-% Cellulose, 5,20 Gew.-% Alkali und 2,3 Gew.-% Schwefel, mit einem Reifeindex von 15 Hottenroth und einer Kugelfallviskosität von 75 Sekunden (bestimmt gemäß dem Zellcheming-Merkblatt III/5/E) wurde mittels einer Spinndüse in ein Regenerierbad, enthaltend 100 g/l Schwefelsäure, 310 g/l Natriumsulfat und 15 g/l Zinksulfat, gesponnen. Die Spinndüse hatte 1053 Löcher mit 50µm Durchmesser. Der Viskosespinnlösung wurden 0,5 Gew.-% einen stickstoffhaltigen Hilfsmittels zugesetzt. Zur Erzielung einer entsprechenden Faserfestigkeit erfolgt eine Verstreckung im Zweitbad (92 C, 15 g/l H2S04) von ca. 75%. Die Abzugsgeschwindigkeit beträgt 50 m/min.

Der Viskoselösung wurden vor der Spinndüse mithilfe einer zwangsfördernden Pumpe verschiedene Gewichtsprozente einer α(1→3)-Glucanlösung (hergestellt mit 5 Gew.-% NaOH, 8 Gew.-% α(1→3)-Glucan) zugesetzt und Fasern mit 5, 10, 15 und 30% Glucan hergestellt (Abbildung 1, V2). Diese Glucan-Mengen beziehen sich auf den Massenanteil des α(1→3)-Glucans an der faserbildenden Substanz.

In einem Vergleichsbeispiel 2 wurde die Viskose aus Beispiel 2 ohne Zusatz der Glucan-NaOH-Lösung, aber unter ansonsten gleichen Bedingungen wie in Beispiel 2 zu Fasern versponnen.

Die Eigenschaften der erhaltenen Fasern sind in der Tabelle 1 angegeben:

### Beispiel 3:

Eine Modal-Viskose, enthaltend 6,0 Gew.-% Cellulose, 6,20 Gew.-% Alkali und 1,8 Gew.-% Schwefel, mit einem Gammawert von 65 und einer Kugelfallviskosität von 130 Sekunden (bestimmt gemäß dem Zellcheming-Merkblatt III/5/E) wurde mittels einer Spinndüse in ein Regenerierbad, enthaltend 72 g/l Schwefelsäure, 115 g/l Natriumsulfat und 55 g/l Zinksulfat, gesponnen. Die Spinndüse hatte 1053 Löcher mit 45µm Durchmesser. Der Viskosespinnlösung wurden 2,5 Gew.-% einen stickstoffhaltigen Hilfsmittels zugesetzt. Zur Erzielung einer entsprechenden Faserfestigkeit erfolgt eine Verstreckung im Zweitbad (92 C, 15 g/l H2S04) von ca. 115%. Die Abzugsgeschwindigkeit beträgt 23 m/min.

Der Viskoselösung wurden vor der Spinndüse mithilfe einer zwangsfördernden Pumpe verschiedene Gewichtsprozente einer α(1→3)-Glucanlösung (hergestellt mit 5 Gew.-% NaOH, 4,5 Gew.-% α(1→3)-Glucan) zugesetzt und Fasern mit 5 und 15 % Glucan hergestellt. Diese Glucan-Mengen beziehen sich auf den Massenanteil des α(1→3)-Glucans an der faserbildenden Substanz.

In einem Vergleichsbeispiel 3 wurde die Viskose aus Beispiel 3 ohne Zusatz der Glucan-NaOH-Lösung, aber unter ansonsten gleichen Bedingungen wie in Beispiel 3 zu Fasern versponnen.

Die Eigenschaften der erhaltenen Fasern sind in der Tabelle 1 angegeben:

**Tabelle 1**

| Beispiel | Zusatz | Glucan-Menge Gew.-% | Titer dtex | FFk cN/tex | FDk % | FFn cN/tex | FDn % |
|---|---|---|---|---|---|---|---|
| VergleichsBeispiel 1 | ohne | - | 1,7 | 27,4 | 16,2 | 15,7 | 16,8 |
| 1a | Glucan DP_{w}800 | 10 | 1,7 | 27,4 | 16,5 | 15,0 | 17,1 |
| 1b | Glucan DP_{w}800 | 25 | 1,7 | 21,9 | 14,2 | 12,0 | 16,3 |
| VergleichsBeispiel 2 | ohne | - | 1,3 | 29,6 | 15,8 | 17,4 | 16,6 |
| 2a | Glucan DP_{w} 800 | 5 | 1,3 | 29,2 | 16,1 | 16,0 | 17,7 |
| 2b | Glucan DP_{w} 800 | 10 | 1,3 | 28,6 | 17,9 | 14,9 | 21,1 |
| 2c | Glucan DP_{w} 800 | 15 | 1,3 | 26,1 | 18,1 | 12,7 | 21,1 |
| 2c | Glucan DP_{w} 800 | 30 | 1,3 | 23,6 | 19,4 | 12,1 | 20,1 |
| VergleichsBeispiel 3 | ohne | - | 1,3 | 38,8 | 12,6 | 22,7 | 13,0 |
| 3a | Glucan DF_{w} 1000 | 5 | 1,3 | 37,6 | 13,3 | 22,1 | 14,3 |
| 3b | Glucan DP_{w} 1000 | 15 | 1,3 | 36,2 | 13,4 | 20,3 | 13,9 |

| | | | | | | | |
|---|---|---|---|---|---|---|---|
| FFk Faserfestigkeit konditioniert FDk Faserdehnung konditioniert FFn Faserfestigkeit nass FDn Faserdehnung nass | | | | | | | |

## Patentansprüche

1. Verfahren zur Herstellung einer Polysaccharid-Faser nach einem Xanthogenatverfahren, **dadurch gekennzeichnet, dass** die faserbildende Substanz eine Mischung aus Cellulose und α(1→3)-Glucan ist.

2. Verfahren nach Anspruch 1, wobei die faserbildende Substanz zwischen 1 und 99 Gew.-%, bevorzugt zwischen 5 und 45 Gew.-% α(1→3)-Glucan enthält.

3. Verfahren nach Anspruch 1, wobei das Verfahren ein Viscoseverfahren ist

4. Verfahren nach Anspruch 1, wobei das Verfahren ein modifiziertes Viscoseverfahren zur Herstellung von Modalfasern ist, in dem das α(1→3)-Glucan in Form einer α(1→3)-Glucan-haltigen Natronlaugelösung zugesetzt wird.

5. Verfahren nach Anspruch 1, wobei das α(1→3)-Glucan zu mindestens 90 % aus Hexose-Einheiten besteht und mindestens 50 % der Hexose-Einheiten durch α(1→3)-glycosidische Bindungen verknüpft sind.

6. Verfahren gemäß den vorhergehenden Ansprüchen, wobei die Faser eine Stapelfaser oder ein Endlosfilament ist.

7. Polysaccharid-Faser, hergestellt nach einem Xanthogenatverfahren, **dadurch gekennzeichnet, dass** sie als faserbildende Substanz Cellulose und α(1→3)-Glucan enthält.

8. Polysaccharid-Faser nach Anspruch 7, wobei die faserbildende Substanz zwischen 1 und 99 Gew.-%, bevorzugt zwischen 5 und 45 Gew.-% α(1→3)-Glucan enthält.

9. Polysaccharid-Faser nach Anspruch 7, wobei das α(1→3)-Glucan zu mindestens 90 % aus Hexose-Einheiten besteht und mindestens 50 % der Hexose-Einheiten durch α(1→3)-glycosidische Bindungen verknüpft sind.

10. Polysaccharid-Faser gemäß den vorhergehenden Ansprüchen, wobei die Faser eine Stapelfaser oder ein Endlosfilament ist.

11. Verwendung der Polysaccharid-Faser nach Anspruch 7 zur Herstellung von textilen Erzeugnissen wie Garnen, Geweben, Gestricken oder Gewirken.

12. Verwendung der Polysaccharid-Faser nach Anspruch 7 zur Herstellung von Vliesstoffen, Hygieneartikeln, insbesondere Tampons, Slipeinlagen und Windeln und sonstigen, absorbierenden Nonwovens-Produkten und Papieren.

13. Verwendung gemäß den Ansprüchen 11 oder 12, wobei die Polysaccharid-Faser eine Stapelfaser oder ein Endlosfilament ist.

## Claims

1. A method for the production of a polysaccharide fiber by using a xanthogenate process, **characterized in that** the fiber-forming substance is a mixture of cellulose and α(1→3)-glucan.

2. The method as claimed in claim 1, wherein the fiber-forming substance contains between 1 and 99% by weight, preferably between 5 and 45% by weight, of α(1→3)-glucan.

3. The method as claimed in claim 1, wherein the method is a viscose process.

4. The method as claimed in claim 1, wherein the method is a modified viscose process for the production of modal fibers in which said α(1→3)-glucan is added in the form of a α(1→3)-glucan-containing sodium hydroxide solution.

5. The method as claimed in claim 1, wherein at least 90% of said α(1→3)-glucan are hexose units and at least 50% of the hexose units are linked via α(1→3)-glycosidic bonds.

6. The method as claimed in any of the preceding claims, wherein the fiber is a staple fiber or a continuous filament.

7. A polysaccharide fiber produced by using a xanthogenate process, **characterized in that** it contains cellulose and α(1→3)-glucan as the fiber-forming substance.

8. The polysaccharide fiber as claimed in claim 7, wherein the fiber-forming substance contains between 1 and 99% by weight, preferably between 5 and 45% by weight, of α(1→3)-glucan.

9. The polysaccharide fiber as claimed in claim 7, wherein at least 90% of said α(1→3)-glucan are hexose units and at least 50% of the hexose units are linked via α(1→3)-glycosidic bonds.

10. The polysaccharide fiber as claimed in any of the preceding claims, wherein the fiber is a staple fiber or a continuous filament.

11. A use of the polysaccharide fiber as claimed in claim 7 for the production of textile products such as yarns, woven fabrics, or knitted fabrics.

12. A use of the polysaccharide fiber as claimed in claim 7 for the production of nonwovens, hygiene articles, particularly tampons, panty liners, and diapers, and of other, absorbent nonwoven products and papers.

13. The use as claimed in claims 11 or 12, the polysaccharide fiber being a staple fiber or a continuous filament.

## Revendications

1. Procédé de fabrication d'une fibre de polysaccharide selon un procédé xanthate de cellulose, **caractérisé en ce que** la substance fibrogène est un mélange de cellulose et de α(1→3)-glucane.

2. Procédé selon la revendication 1, la substance fibrogène contenant entre 1 et 99 % en masse, de préférence entre 5 et 45 % en masse de α(1→3)-glucane.

3. Procédé selon la revendication 1, le procédé étant un procédé viscose.

4. Procédé selon la revendication 1, le procédé étant un procédé viscose modifié pour la fabrication de fibres de modal dans lequel du α(1→3)-glucane est ajouté sous la forme d'une solution de soude caustique contenant du α(1→3)-glucane.

5. Procédé selon la revendication 1, le α(1→3)-glucane étant constitué au moins à 90 % d'unités d'hexose, et 50 % des unités d'hexose étant liées par des liaisons glycosidiques en α(1→3).

6. Procédé selon les revendications précédentes, la fibre étant une fibre discontinue ou un filament continu.

7. Fibre de polysaccharide fabriquée selon un procédé xanthate de cellulose, **caractérisée en ce qu'**elle contient comme substance fibrogène de la cellulose et du α(1→3)-glucane.

8. Fibre de polysaccharide selon la revendication 7, la substance fibrogène contenant entre 1 et 99 % en masse, de préférence entre 5 et 45 % en masse de α(1→3)-glucane.

9. Fibre de polysaccharide selon la revendication 7, le α(1→3)-glucane étant constitué au moins à 90 % d'unités d'hexose, et 50 % des unités d'hexose étant liées par des liaisons glycosidiques en α(1→3).

10. Fibre de polysaccharide selon les revendications précédentes, la fibre étant une fibre discontinue ou un filament continu.

11. Utilisation de la fibre de polysaccharide selon la revendication 7 pour la fabrication de produits textiles comme des fils, tissus ou tricots.

12. Utilisation de la fibre de polysaccharide selon la revendication 7 pour la fabrication de non-tissés, d'articles d'hygiène, en particulier de tampons, de protège-slip, de couches et autres produits non-tissés et papiers absorbants.

13. Utilisation selon les revendications 11 ou 12, la fibre de polysaccharide étant une fibre discontinue ou un filament continu.
